# EUROPEAN PATENT SPECIFICATION

(11) **EP 2 353 555 B1**
(45) Date of publication and mention of the grant of the patent: **16.09.2015**
(21) Application number: 11152521.8
(22) Date of filing: 28.01.2011
(51) Int. Cl.: A61F 5/56, A61M 16/04

(54) **Automated negative pressure oral apparatus**
Automatische orale Unterdruckanwendung
Appareil oral à pression négative automatique

(30) Priority: 06.02.2010 US 302091 P
(43) Date of publication of application: 10.08.2011
(73) Proprietor: Somnics Inc., Zhubei Hsin chu 30274 (TW)
(72) Inventor: Chen, Chung-Chu, 302, Hsin-Chu (TW)
(74) Representative: Charrier, Rapp & Liebau

(56) References cited:
- EP-A1- 2 347 739
- WO-A1-99/61089
- WO-A2-2007/075491
- US-A- 4 114 626

## Description

### BACKGROUND OF THE INVENTION

### 1. Field of the Invention

The present invention is related to an oral apparatus of keeping mouth closed and applying negative pressure through a mini oral interface to the oral cavity. More particularly, the mini oral interface creates a secure connection to mouth and helps a user's upper and lower lips closed during sleeping.

### 2. Prior Art

Obstructive sleep apnea (OSA) is a condition in which repeated collapses in the patient's airway during inhalation causes a cessation of breathing during sleep. During inhalation, air pressure in the lungs and respiratory passages is reduced. If during this time, the tone of the muscles in the upper-airway is reduced, the airway tends to collapse. As the airway begins to occlude prior to an apnea episode, the patient often begins to snore. Snoring is an effort to try to combat the collapsed airway. These obstructions occur in different locations along the respiratory pathway in different patients, but the two common locations are the oropharynx or the nasopharynx.

People with moderate to severe OSA experience daytime sleepiness, fatigue, and poor concentration. In addition to these immediate problems, research has shown that patients with OSA use more medical resources, have an increased risk of medical disability, and finally have a higher mortality rate. Patients with severe OSA are estimated to have a three to six fold increased risk of mortality considering all causes. OSA is also implicated in many cardiovascular conditions, such as systemic hypertension and some degree of pulmonary hypertension. It is associated with an increased risk for myocardial infarction, cerebrovascular disease, and cardiac arrhythmia. OSA causes excessive daytime sleepiness due to interrupted sleeping pattern at night which leads to inability to concentrate. Patients' daily functions are impaired as their neuro-cognitive function is compromised. They are more likely to make errors and run into accidents. Therefore, OSA is a significant medical condition with serious negative outcomes if left untreated.

There are several current treatment options for OSA patients. Oral appliances are used to treat mild OSA, but they often don't work well and cause damage to gums and teeth. Several types of surgery are used to treat OSA, however, surgical options are invasive, expensive and painful with recovery periods up to 6 months. The most common treatment for moderate to severe sleep apnea in adults is CPAP, which has 96% market share in OSA therapeutics. A CPAP machine consists of a mask, a pump and a humidifier. The device continuously blows pressurized air into the patient's nose to keep the airway open during sleep. CPAP is quite effective; however, it has unpleasant side effects such as dry throat and nose congestion. Patients who use CPAP often feel bloated in the morning and experience headaches. The machine is noisy and uncomfortable for the user and their partner. CPAP is currently the first-line and gold standard treatment, but it suffers low compliance due to significant side effects.

It has been proposed to apply a negative pressure to the patient's oral cavity to pull the tongue and soft palate forward to maintain the patency of the airway, as an improvement over CPAP, for example, U.S. Patent and Patent Publication Nos. 5957133, 2005/0166928, and 2006/0096600. While promising in theory, these prior arts comprise of relatively large structures to engage the teeth and/or to retain the tongue. Moreover, negative pressure is applied directly on the soft tissues of the tongue to hold the tongue within the cavity. These approaches tend to occupy a lot of space in the oral cavity, which may cause discomfort and damage to large area of teeth, gum, and soft tissues. At the same time, the presence of such larger devices may induce excess saliva secretion and elicit the gag reflex. The other major disadvantage of these approaches is that the oral devices are anatomically dependent, requiring special technicians to customize the interface for each individual patient.

US 4 116 626 A discloses a adhesive patch arrangement including upper and lower adhesive patches rotatably connected together by an attaching structure inter alia comprising a retaining ring and a passage for a tube for insertion into patient's mouth. However, the two patches cannot be disconnected or moved in a direction parallel to a direction from the upper lip toward the lower lip, so the patient will not be able to open the mouth.

EP 2 347 739 A1 (Article 54(3) EPC document) discloses an oral apparatus and method capable of alleviating or curing snore and obstructive sleep apnea by applying a negative pressure through a mini oral interface to the oral cavity. The mini oral interface creates a secure connection to mouth and prevents disengaging from patient's mouth during sleeping. The negative pressure pulls the tongue toward upper palate and also pulls the soft palate forward as well. By moving the tongue and the soft tissue in a forward direction, the patency of the upper airway near the pharynx is maintained to prevent sleep-disordered breathing. The negative pressure will pull the lips inward to close the mouth preventing air from entering the oral cavity from atmosphere. The negative pressure will also pull the soft palate into contact with the rear surface of the tongue to create a seal that prevents the air from entering the oral cavity through the nasal airway.

Therefore, it is one objective of the present invention to provide alternative and improved apparatus significantly improve patency of a patient's airway during sleep.

### SUMMARY OF THE INVENTION

The present invention provides an oral apparatus for improving airway patency according to claim 1. Preferred embodiments are disclosed in dependent claims 2-15.

The present invention provides an oral apparatus, comprising: an upper component and a lower component, which can be temporarily attached to the skin around upper lip and lower lip separately; a connecting part to connect and/or disconnect, as well as to control a distance between the upper and lower components; a retractable part, which connects to the connecting part and may exert a pulling force to close the lips and mouth. The upper and lower components may only be applied to lip region away from the opening of the mouth to allow for the mouth to open partially. The retractable part not only provides a mechanism to close a user's mouth, meanwhile, it may also provide a mechanism to allow the user to open the mouth at certain conditions (such as coughing, sneezing, and yawning, etc.). The oral apparatus may further connect to a negative pressure source and automatically detect if the user's mouth is open or other physiological conditions such as sleep stages. If the user's mouth is open, the retractable part may voluntarily close the user's mouth and prevent it from opening. The negative pressure may only be applied when the user's mouth is closed so that the treatment can be more efficient, quiet, and comfortable for the users. The oral apparatus can also be used in combination with constant positive airway pressure devices, oral appliances, or other sleep apnea therapies to prevent mouth breathing.

In the present invention the connecting part is a flexible wire and the flexible wire is connected to an anchoring part on the upper attaching component.

In another embodiment, the present invention provides an oral apparatus, wherein the retractable part is a piston-type retractable part.

An example provides an oral apparatus, wherein the retractable part is a bellows-type retractable part.

Another example provides an oral apparatus, wherein the retractable part is a magnetic retractable part.

Another example provides an oral apparatus, wherein the retractable part is a spring-type retractable part.

Another example provides an oral apparatus, wherein the retractable part is a plate-type retractable part.

Another example provides an oral apparatus, wherein the retractable part is a self-retracting-drum-type retractable part.

Yet another example, an oral apparatus, comprising: an upper component and a lower component, which can be temporarily attached to the skin around upper lip and lower lip separately; an active connecting part to connect and/or disconnect as well as to control a distance between the upper and lower components. The active connecting part may further comprise an actuator and a controller.

### BRIEF SUMMARY OF THE DRAWINGS

The objects and advantages of the preferred embodiments of the present invention will be readily understood by the accompanying drawings and detailed descriptions, wherein:
Fig. 1A and Fig. 1B show schematic diagrams with front views of an oral apparatus according to the first embodiment;
Fig. 1C to Fig 1I show schematic diagrams of different retractable parts may be adopted in the first embodiment.
Fig. 2A and Fig. 2B show schematic diagrams with front views of an oral apparatus according to Fig. 1A and Fig. 1B being used in combination with a conduit and a negative pressure source;
Fig. 3 shows a schematic diagram with a front view of an oral apparatus according Fig. 2A and Fig. 2B further comprising an extensible link(s) between the upper and lower attaching components;
Fig. 4A and Fig. 4B show schematic diagrams with front views of an oral apparatus according to the second embodiment;
Fig. 4C and Fig. 4D show schematic diagrams of actuators and controllers of an active connecting part according to Fig. 4A and Fig. 4B;
Fig. 5A to Fig. 5D show schematic diagrams with front views of an oral apparatus according to Fig. 4A to Fig. 4D being used in combination with a conduit and a negative pressure source;
Fig. 6 to Fig. 12 show schematic diagrams of variations of attaching components of an oral apparatus according to Fig 5A and Fig. 5B;
Fig. 13A shows a perspective diagram of the oral apparatus according to of Fig. 6 with an active connecting part disconnected;
Fig. 13B shows a perspective diagram of the oral apparatus according to Fig. 6 with an active connecting part connected in a mouth-closed condition;
Fig. 13C shows a perspective diagram of the oral apparatus according to Fig. 6 with an active connecting part connected in a mouth-open condition;
Fig. 14 shows a perspective diagram of the oral apparatus according to Fig. 13 further comprising an extensible link(s);
Fig. 15 shows a perspective diagram of the oral apparatus according to Fig. 13 further comprising a sensor(s) to detect physiological conditions;
Fig. 16 shows a perspective diagram of the oral apparatus according to Fig. 13 further comprising a liquid collector;
Fig. 17 shows a perspective exploded diagram of the liquid collector according to Fig. 16;
Fig. 18 shows the liquid collector according to Fig. 16, wherein the left side view shows a cross-section view of the liquid collector and the right side view shows a fragmentarily cross-section view of the liquid collector;
Fig. 19 shows a perspective diagram of the liquid collector according to Fig. 16 further comprising a filter valve and/or a sensor(s);
Fig. 20 shows a flow chart of a method not part of the invention according to the second embodiment of the present invention for automated control of negative pressure pumping according to whether a user's mouth is open or closed.

### DETAILED DESCRIPTION OF THE INVENTION

Reference will now be made in detail to the present exemplary embodiments, examples of which are illustrated in the accompanying drawings. Wherever possible, the same reference numbers are used in the drawings and the description to refer to the same or like parts.

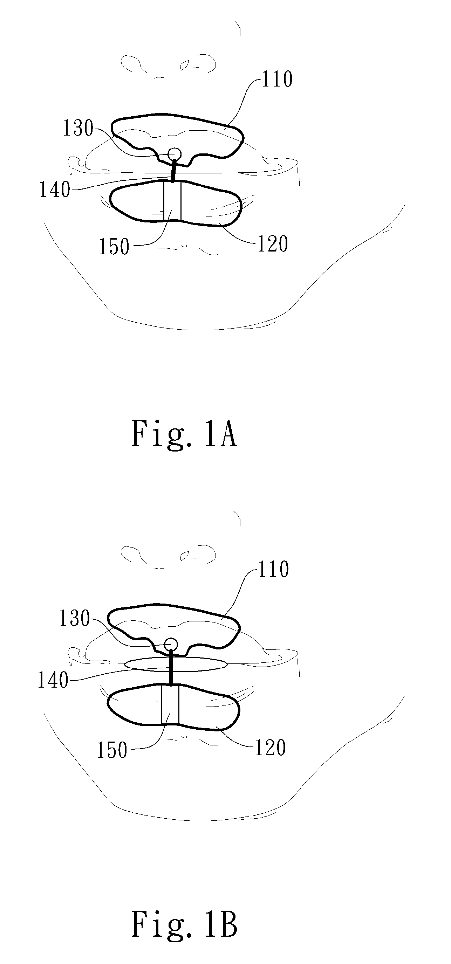

Fig. 1A and Fig. 1B show schematic diagrams of an oral apparatus according to the first embodiment.

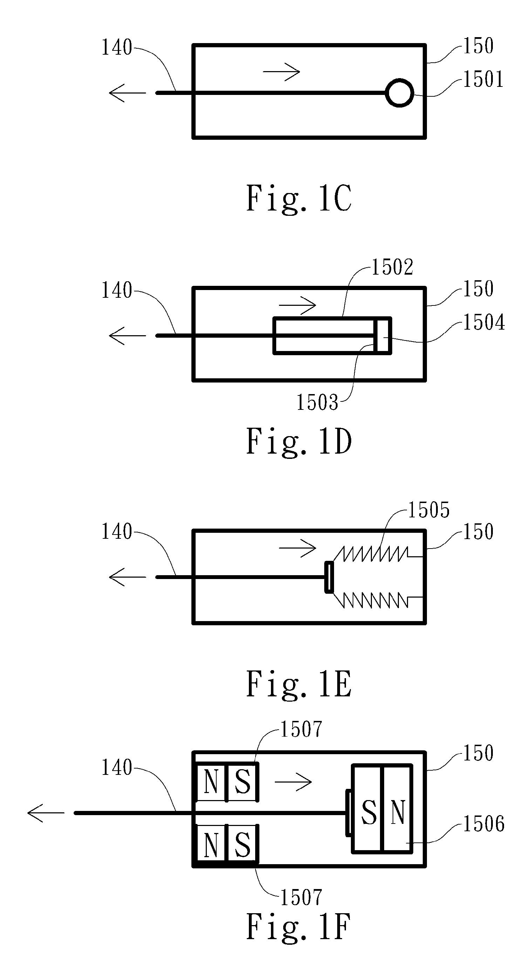

Fig. 1A demonstrates the oral apparatus with a user's mouth closed and Fig. 1B with a user's mouth open. The oral apparatus comprises an upper attaching component 110, a lower attaching component 120, an anchoring part 130, a connecting part 140, and a retractable part 150. The attaching components 110 and 120 can be of various thickness and flexibility, and is capable of adhering firmly to the skin. Potential materials of the attaching components include silicone, flexible plastic, latex, gel, hydrogel, acrylic gel as well as other materials coated with an adhesive substance. The adhesive upper and lower attaching components 110 and 120 can be temporarily attached to the skin around the upper and lower lips, respectively. The anchoring part 130 can be attached or fixed on the upper attaching component 110, and the retractable part 150 can be attached or fixed on the lower attaching component 120. The connecting part 140 has two ends connecting to the anchoring part 130 and retractable part 150, respectively. Fig. 1C shows a schematic diagram of the connecting part 140 connecting to the retractable part 150, wherein the retractable part 150 is a fixed retractable part and the connecting part 140 is an extensible connecting part. The fixed retractable part 150 comprises an anchor 1501. The anchor 1501 is fixed in the retractable part 150, i.e., the position of the anchor 1501 is unchangeable relative to the fixed retractable part 150. The extensible connecting part 140 is elastic and its length can be changed. Please refer to Fig. 1A and Fig. 1B, in the initial condition, the length of the extensible connecting part 140 can maintain a user's mouth closed.

At certain conditions when the user needs to open the mouth (such as coughing, sneezing, and yawning, etc.), upper and lower lips apply a pulling force via the anchoring part 130 and the retractable part 150, respectively, to stretch the length of the extensible connecting part 140. Therefore, the distance between the anchoring part 130 and the retractable part 150 increases and the user's mouth can be opened. After the specific conditions (such as sneezing) finish, the upper and lower lips stop applying a pulling force via the anchoring part 130 and the retractable part 150, respectively, to the extensible connecting part 140, the extensible part 140 can restore its initial length due to its elastic property. Therefore, the extensible connecting part 140 applies a force (restoring force) to the upper and lower lips to close the user's mouth. In the first embodiment of the present invention, the connecting part 140 and the retractable part 150 can have different variations to provide a mechanism to allow a user to open the mouth when the user needs; when the user does not need to open the mouth, the oral apparatus helps close the user's mouth.

Fig. 1D is a variation of the connecting part 140 and retractable part 150 according to the first embodiment.

The retractable part 150 is a piston-type retractable part, which comprises a cylinder 1502 and a piston 1503. The connecting part 140 has a fixed length and is connected to the piston 1503. Please refer to Fig. 1A and Fig. 1B, in the initial condition, the position of the piston 1503 and the length of the connecting part 140 can maintain a user's mouth closed. The piston 1530 is kept at a balanced position and divided the cylinder 1502 into two, left and right, isolated compartments. At certain conditions when the user needs to open the mouth (such as coughing, sneezing, and yawning, etc.), the connecting part 140 pulls the piston 1503 toward the left side, as shown by the arrow direction of Fig. 1D. As a consequence, the pressure in the right compartment 1504 decreases, and the pressure in the left compartment increases. Therefore, the distance between the anchoring part 130 and the retractable part 150 increases and the user's mouth can be opened. After the specific conditions (such as sneezing) finish, the connecting part 140 stops pulling the piston 1503 toward left. As the pressure in the left compartment is higher than the pressure in the right compartment 1504, the piston 1503 is pushed back to the original balanced position. Therefore, the extensible connecting part 140 is pulled back to its initial conditions and keeps the user's mouth closed.

Fig. 1E is another variation of the connecting part 140 and retractable part 150 according to the first embodiment.

The retractable part 150 is a bellows-type retractable part, which comprises a bellows 1505. The connecting part 140 has a fixed length and is connected to the bellows 1505. Please refer to Fig. 1A and Fig. 1B, in the initial condition, the position of one end of the bellows 1505 and the length of the connecting part 140 can maintain a user's mouth closed. At certain conditions when the user needs to open the mouth (such as coughing, sneezing, and yawning, etc.), the connecting part 140 pulls the one end of the bellows 1505 toward the left side, as shown by the arrow direction of Fig. 1E. As a consequence, the distance between the anchoring part 130 and the bellows-type retractable part 150 increases and the user's mouth can be opened. After the specific conditions (such as sneezing) finish, the connecting part 140 stops pulling the one end of the bellows 1505 toward left. As the bellows 1505 tends to return to its initial volume, i.e., the one end of the bellows 1505 tends to return to its initial position, the connecting part 140 is pulled back toward right to its initial conditions and keeps the user's mouth closed.

Fig. 1F is yet another variation of the connecting part 140 and retractable part 150 according to the first embodiment.

The retractable part 150 is a magnetic-type retractable part, which comprises a first magnetic part 1506 and a second magnetic part 1507. The connecting part 140 has a fixed length and is connected to the first magnetic part 1506. Please refer to Fig. 1A and Fig. 1B, in the initial condition, the position of the first magnetic part 1506 and the length of the connecting part 140 can maintain a user's mouth closed. At certain conditions when the user needs to open the mouth (such as coughing, sneezing, and yawning, etc.), the connecting part 140 pulls the first magnetic part 1506 toward the left side, as shown by the arrow direction of Fig. IF. As a consequence, the distance between the anchoring part 130 and the magnetic-type retractable part 150 increases and the user's mouth can be opened. After the specific conditions (such as sneezing) finish, the connecting part 140 stops pulling the first magnetic part 1506 toward left. As the second magnetic part 1507 pushes the first magnetic part 1506 back to its initial position, the connecting part 140 is pulled back toward right to its initial conditions and keeps the user's mouth closed.

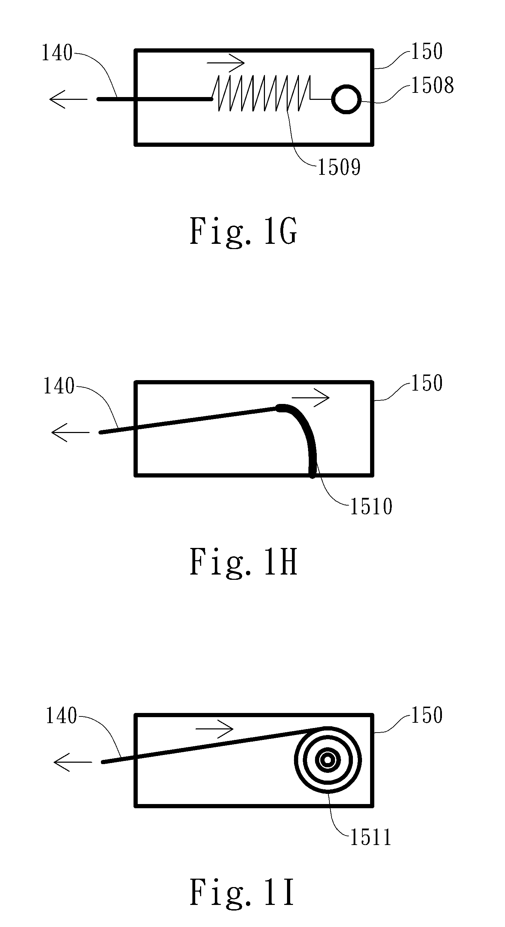

Fig. 1G is yet another variation of the connecting part 140 and retractable part 150 according to the first embodiment.

The retractable part 150 is a spring-type retractable part, which comprises an anchor 1508 and a spring 1509. The spring 1509 has a first end and a second end, wherein the first end connects to the connecting part 140 and the second end connects to the anchor 1508. The connecting part 140 has a fixed length. Please refer to Fig. 1A and Fig. 1B, in the initial condition, the position of the first end of the spring 1509 and the length of the connecting part 140 can maintain a user's mouth closed. At certain conditions when the user needs to open the mouth (such as coughing, sneezing, and yawning, etc.), the connecting part 140 pulls the first end of the spring 1509 toward the left side, as shown by the arrow direction of Fig. 1G. As a consequence, the distance between the anchoring part 130 and the spring-type retractable part 150 increases and the user's mouth can be opened. After the specific conditions (such as sneezing) finish, the connecting part 140 stops pulling the first end of the spring 1509 toward left. As the spring 1509 tends to return to its initial length, i.e., the first end of the spring 1509 tends to return to its initial position, the connecting part 140 is pulled back toward right to its initial conditions and keeps the user's mouth closed.

Fig. 1H is still another variation of the connecting part 140 and retractable part 150 according to the first embodiment.

The retractable part 150 is a plate-type retractable part, which comprises a plate 1510. The plate 1510 has a first end and a second end, wherein the first end connects to the connecting part 140 and the second end connects to a fixed position of the plate-type retractable part 150. The connecting part 140 has a fixed length. Please refer to Fig. 1A and Fig. 1B, in the initial condition, the position of first end of the plate 1510 and the length of the connecting part 140 can maintain a user's mouth closed. At certain conditions when the user needs to open the mouth (such as coughing, sneezing, and yawning, etc.), the connecting part 140 pulls the first end of the plate 1510 toward the left side, as shown by the arrow direction of Fig. 1H. As a consequence, the distance between the anchoring part 130 and the plate-type retractable part 150 increases and the user's mouth can be opened. After the specific conditions (such as sneezing) finish, the connecting part 140 stops pulling the first end of the plate 1510 toward left. As the plate 1510 tends to return to its initial condition, i.e., the first end of the plate 1510 tends to return to its initial position, the connecting part 140 is pulled back toward right to its initial conditions and keeps the user's mouth closed.

Fig. 1I is yet still another variation of the connecting part 140 and retractable part 150 according to the first embodiment.

The retractable part 150 is a self-retracting-type retractable part, which comprises a self-retracting drum 1511. The connecting part 140 has a fixed length and is connected to the self-retracting drum 1511. Please refer to Fig. 1A and Fig. 1B, in the initial condition, the position of one end of the self-retracting drum 1511 and the length of the connecting part 140 can maintain a user's mouth closed. At certain conditions when the user needs to open the mouth (such as coughing, sneezing, and yawning, etc.), the connecting part 140 pulls the one end of the self-retracting drum 1511 toward the left side, as shown by the arrow direction of Fig. 1I. As a consequence, the distance between the anchoring part 130 and the self-retracting-type retractable part 150 increases and the user's mouth can be opened. After the specific conditions (such as sneezing) finish, the connecting part 140 stops pulling the one end of the self-retracting drum 1511 toward left. As the self-retracting drum 1511 tends to return to its initial condition, i.e., the one end of the self-retracting drum 1511 tends to return to its initial position, the connecting part 140 is pulled back toward right to its initial conditions and keeps the user's mouth closed.

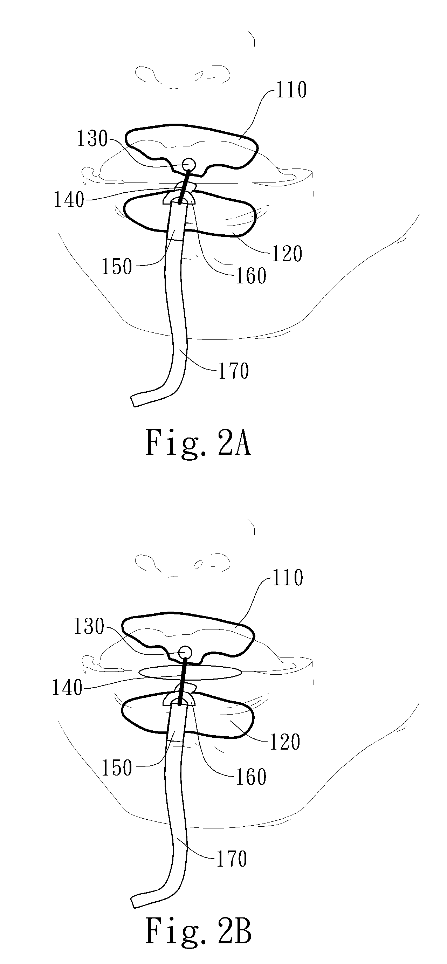

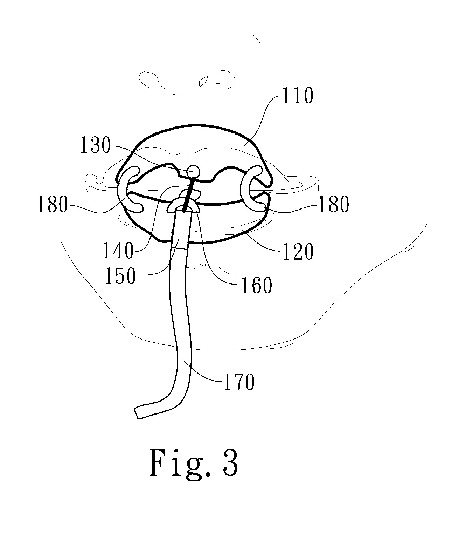

Fig. 2A and Fig. 2B demonstrate the front views of an oral apparatus according to Fig. 1A and Fig. 1B being used as a negative pressure sleep apnea treatment device. The oral apparatus further comprises a fluid conduit 170. The first end of the fluid conduit 170 is attached to the lower attaching component 120 and enters a user's oral cavity. The second end of the fluid conduit 170 is connected to a negative pressure source (not shown). The negative pressure source can draw air out of oral cavity and thus produce a negative pressure environment to pull the tongue, soft palate and other soft tissue forward to maintain the airway patency reducing snoring and apnea episodes. The oral apparatus can further comprise a fluid conduit attachment part 160. The fluid conduit attachment part 160 is used to attach the fluid conduit 170 on the lower attaching component 120. Please notice that the retractable part 150 can be installed on the fluid conduit 170 or the lower attaching component 120. In one preferred embodiment of the present invention, the anchoring part 130 can have a design to allow the upper attaching component 110 to separate with the lower attaching component 120, as described in the following section. Therefore, the oral apparatus can further comprise an extensible link(s) 180. The extensible link is flexible and can connect the upper attaching component 110 and the lower attaching component 120 to prevent loss of any of the attaching components due to separation, as shown in Fig. 3.

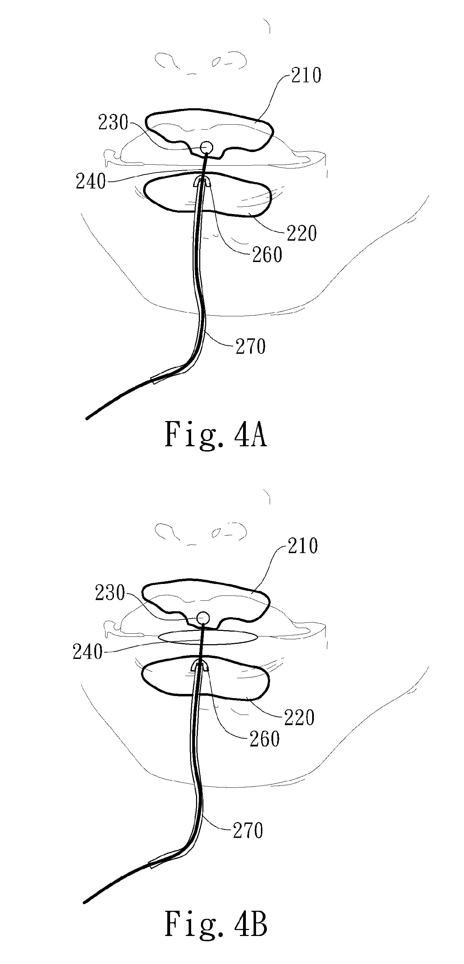

Fig. 4A and Fig. 4B are schematic diagrams with front views of an oral apparatus according to the second embodiment.

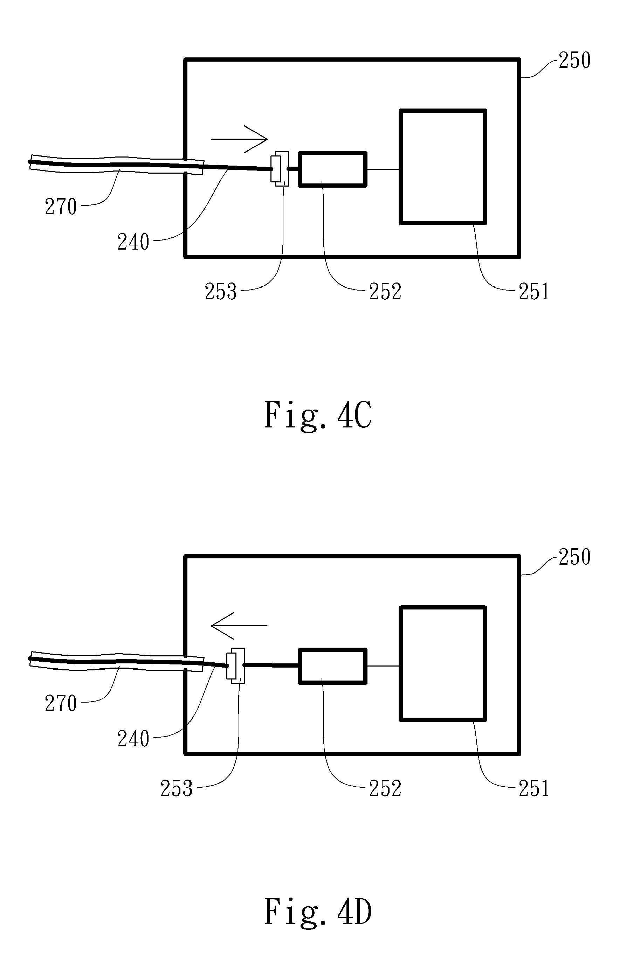

Fig. 4A is the schematic diagram with a user's mouth closed and Fig 4B with a user's mouth open. The oral apparatus comprises of an upper attaching component 210, a lower attaching component 220, an anchoring part 230, a connecting part 240, and a retractable part 250 (as shown in Fig. 4C). The upper and lower attaching components 210 and 220 can be of various thickness and flexibility, and is capable of adhering firmly to the skin. Potential materials of the attaching components include silicone, flexible plastic, latex, gel, hydrogel, acrylic gel as well as other materials coated with an adhesive substance. The adhesive upper and lower attaching components 210 and 220 can be temporarily attached to the skin around the upper and lower lips, respectively. The anchoring part 230 can be attached or fixed on the upper attaching component 210. The connecting part 240 is connected to the anchoring part 230 on one end and connected to the retractable part 250 on the other end. Fig. 4C and Fig. 4D are schematic diagrams of the connecting part 240 formed of a wire 240 being connected to the retractable part 250. Fig. 4C is the schematic diagram with a user's mouth closed and Fig 4D with a user's mouth open. The retractable part 250 further comprises a controller 251 and an actuator 252. The connecting part 240 connects to the retractable part 250 via an engaging point 253. The controller 251 determines the conditions and activates a motion of the actuator 252 to apply an inward pulling force via the connecting part 240 on a user's upper lip in order to close the user's mouth (as shown in Fig. 4A and Fig. 4C). The controller 251 may also activate another motion of the actuator 252 to apply an outward pushing force on a user's upper lip in order to open the user's mouth. Please notice that in the second embodiment of the present invention, the connecting part 240 has to be a rigid material that the actuator 252 can pull it inwardly or push it outwardly to adjust the distance between the anchoring part 230 and the retractable part 250, and thus can close or open the user's mouth. Please refer to Fig. 4A. In the second embodiment of the present invention, the oral apparatus further comprises a connecting catheter 270. The connecting part 240 connects to the retractable part 250 via the connecting catheter 270 to prevent the connecting part 240 having contact or friction with a user's face. Moreover, the oral apparatus may further comprise a connecting catheter attachment part 260. The connecting catheter attachment part 260 is used to attach the connecting catheter 270 to the lower attaching component 220.

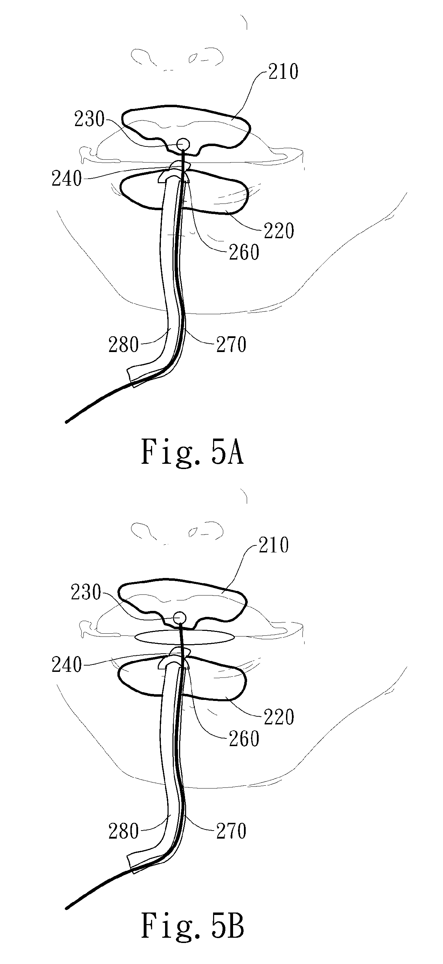

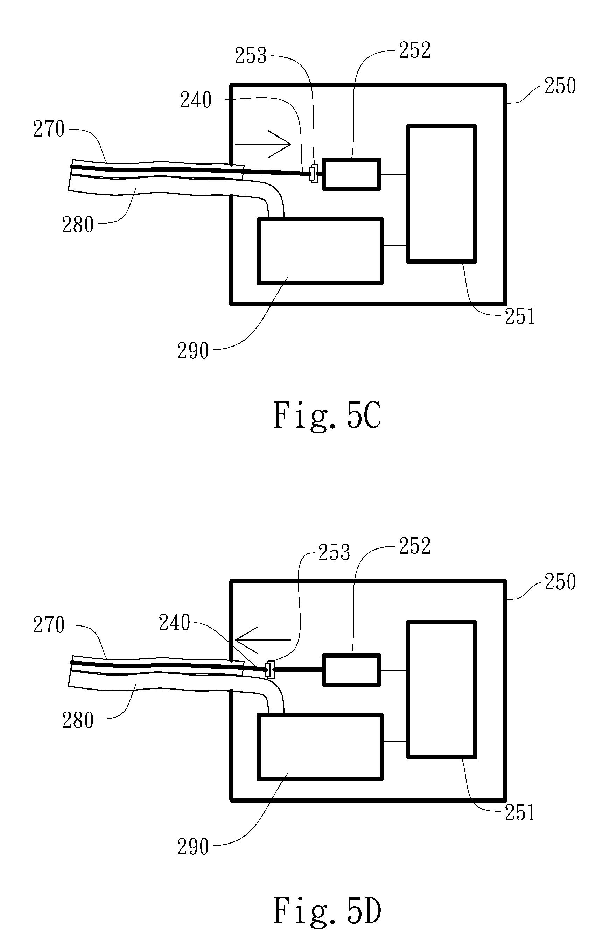

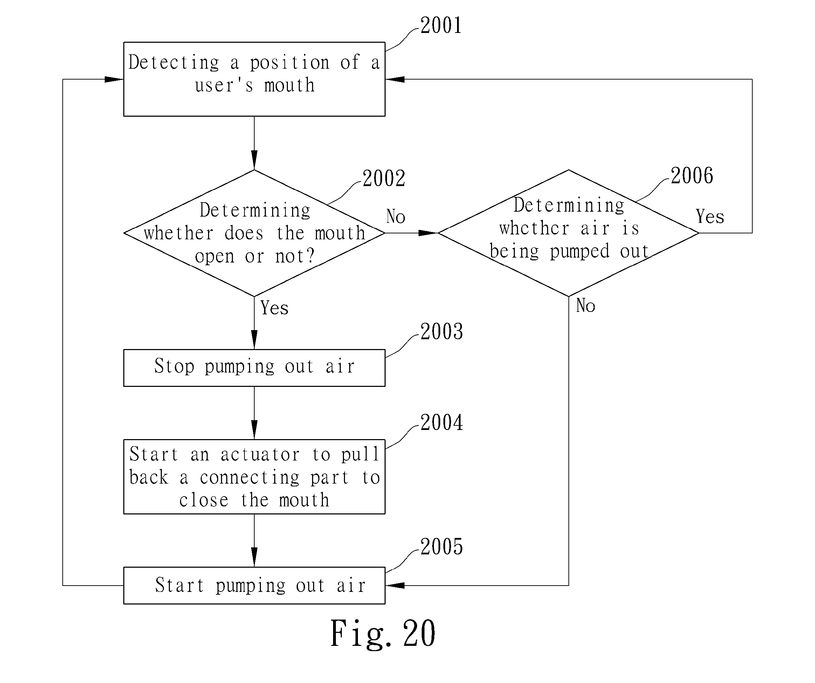

Fig. 5A and Fig. 5B demonstrate the front views of an oral apparatus according to Fig. 4A and Fig. 4B being used as a negative pressure sleep apnea treatment device. The oral apparatus further comprises a fluid conduit 280. The first end of the fluid conduit 280 is attached to the lower attaching component 220 and enters a user's oral cavity. The second end of the fluid conduit 280 is connected to a negative pressure source 290 (not shown). The negative pressure source 290 can draw air out of oral cavity and thus produce a negative pressure environment to pull the tongue, soft palate and other soft tissue forward to maintain the airway patency reducing snoring and apnea episodes. In one preferred embodiment of the present invention, the negative pressure source 290 can be integrated with the retractable part 250 and the controller 251 can be connected with the negative pressure source 290 to control on and off of the negative pressure source 290, as shown in Fig. 5C and Fig. 5D. Please refer to Fig. 5A and Fig. 5B, the connecting catheter 270 and the fluid conduit 280 can be integrated as one piece. The integral piece of the fluid conduit 280 and the connecting catheter 270 can be attached to the lower attaching component 220 via the fluid conduit attachment part 260. In one preferred embodiment of the present invention, the anchoring part 230 can have a design to allow the upper attaching component 210 to separate with the lower attaching component 220, as described in the following section. Therefore, the oral apparatus can further comprise an extensible link(s) (as shown by 180 in Fig. 3). The extensible link is flexible and can connect the upper attaching component 210 and the lower attaching component 220 to prevent loss of any of the attaching components due to separation. Please further refer to Fig. 20 accompanying Fig. 5A to Fig. 5D.

Fig. 20 is a flow chart of a method which automatically applies negative pressure to a user's oral cavity. The anchoring part 230 is installed or fixed on the upper attaching component 210. The connecting part 240 has two ends being connected to the anchoring 230 and the engaging point 253, respectively. In addition, when the actuator 252 pulls the engaging point 253 inwardly (i.e., pulling the connecting part 240 inwardly), a user's mouth can be closed. When the actuator 252 pushes the engaging point 253 outwardly (i.e., pushing the connecting part 240 outwardly), the user's mouth can be opened. When the relative distance d between the engaging point 253 and the actuator 252 is equal to or larger than a given distance d1, i.e. d≥d1, it represents a user's mouth is open. When the relative distance d between the engaging point 253 and the actuator 252 is equal to or smaller than another given distance d2, i.e. d≤d2, it represents a user's mouth is closed. In one embodiment of the present invention, the controller 251 and other external components can detect the relative distance d between the engaging point 253 and the actuator 252, which is equivalent to detect a relative distance between a user's upper lip and lower lip (step 2001), and can determine if the user's mouth is open (step 2002). If the user's mouth is open, i.e. d≥d1 is detected, the controller 251 can turn off the negative pressure source 290 (step 2003), and activate the actuator 252 to pull the connecting part 240 inwardly in order to close the user's mouth, i.e. until d≤d2 is detected; then the controller 251 can turn on the negative pressure source 290. If the user's mouth is closed, i.e. d≤d2 is detected, the controller 251 can check if the negative pressure source 290 is turned on (step 2006); if the negative pressure source 290 is not turned on, the controller 251 can turn on the negative pressure source 290. With the above-mentioned process, a user's mouth can be automatically kept close and applied with negative pressure in the user's oral cavity.

As mentioned above, in the first and second embodiments, various designs of the anchoring parts 130 and 230 can allow the upper attaching components 110 and 120 to be separated from the lower attaching components 120 and 220. This also allows a user to temporarily disengage the anchoring parts 130 or 230 to let the user to open mouth to drink, speak or perform other activities.

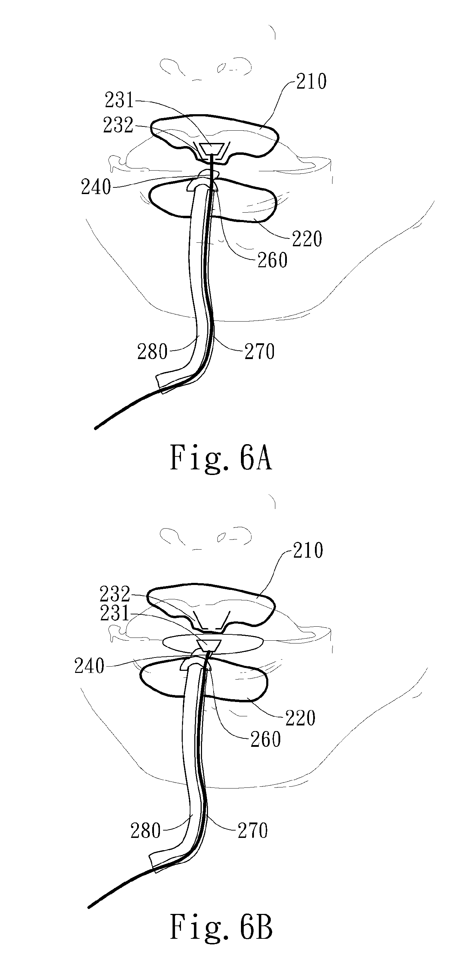

Fig. 6A is a schematic diagram of the oral apparatus according to Fig. 5A wherein the anchoring part 230 is a mechanical anchoring part. The mechanical anchoring part comprises an anchoring block 231 and an anchoring seat 232. The anchoring seat 232 is installed and fixed on the upper attaching component 210, and the anchoring block 231 is connected to the connecting part 240. The anchoring block 231 and the anchoring seat 232 can be joined and disjoined, which allows a user to manually disengage the mechanical anchoring part to let the user to open mouth to drink, speak or perform other activities, as shown in Fig. 6B.

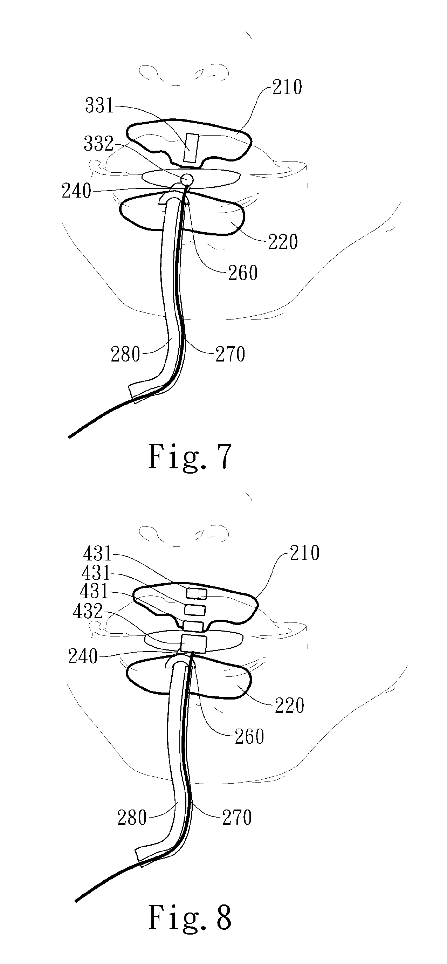

Fig. 7 is a schematic diagram of the oral apparatus according to Fig. 5A wherein the anchoring part 230 is a magnetic anchoring part. The magnetic anchoring part comprises a first magnetic sub-part 331 and a second magnetic sub-part 332. The first magnetic sub-part 331 is installed and fixed on the upper attaching component 210, and the second magnetic sub-part 332 is connected to the connecting part 240. The first magnetic sub-part 331 and the second magnetic sub-part 332 can be joined and disjoined, which allows a user to manually disengage the magnetic anchoring part to let the user to open mouth to drink, speak or perform other activities. Besides, the second magnetic sub-part 332 can be a strip having a longitudinal magnetic connection region with infinite joining points to allow for anatomical difference. It can be adjusted by users to accommodate thicker or thinner lips or provide tighter or looser mouth closing as desired. Either of the above mentioned magnetic sub-parts 331 and 332 can be made of paramagnetic materials.

Fig. 8 is a schematic diagram of the oral apparatus according to Fig. 5A wherein the anchoring part 230 is another mechanical anchoring part. The mechanical anchoring part comprises a male connector 431 and a female connector 432. The male connector 431 is installed and fixed on the upper attaching component 210, and the female connector 432 is connected to the connecting part 240. The male connector 431 and the female connector 432 can be joined and disjoined, which allows a user to disengage the mechanical connector temporarily and manually to let the user to open mouth to drink, speak or perform other activities. Besides, the male connector 431 can have a plural of male connector sub-parts to allow for anatomical difference. It can be adjusted by users to accommodate thicker or thinner lips or provide tighter or looser mouth closing as desired.

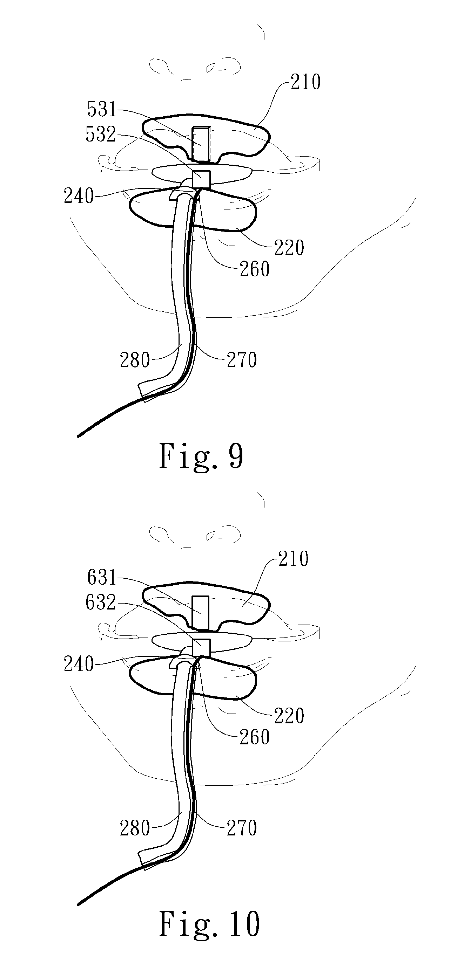

Fig. 9 is a schematic diagram of the oral apparatus according to Fig. 5A wherein the anchoring part 230 is a frictional anchoring part. The frictional anchoring part 230 may comprise a sunken structure 531 and a protruding structure 532. The sunken structure 531 is installed and fixed on the upper attaching component 210, and the protruding structure 532 is connected to the connecting part 240. The sunken structure 531 and the protruding structure 532 can be joined and locked by frictional forces and can also be disjoined by overcoming the frictional forces, which allows a user to disengage the frictional anchoring part temporarily and manually to let the user to open mouth to drink, speak or perform other activities. Besides, the protruding structure 532 can be inserted into different locations on the sunken structure 531 to allow for anatomical difference. It can be adjusted by users to accommodate thicker or thinner lips or provide tighter or looser mouth closing as desired.

Fig. 10 is a schematic diagram of the oral apparatus according to Fig. 5A wherein the anchoring part 230 is an adhesive anchoring part. The adhesive anchoring part 230 may comprise a first adhesive part 631 and a second adhesive part 632. The first adhesive part 631 is installed and fixed on the upper attaching component 210, and the second adhesive part 632 is connected to the connecting part 240. The first adhesive part 631 and the second adhesive part 632 can be attached and detached, which allows a user to separate the adhesive anchoring part temporarily and manually to let the user to open mouth to drink, speak or perform other activities. Besides, the first adhesive part 631 can be attached onto different locations of the second adhesive part 632 to allow for anatomical difference. It can be adjusted by users to accommodate thicker or thinner lips or provide tighter or looser mouth closing as desired.

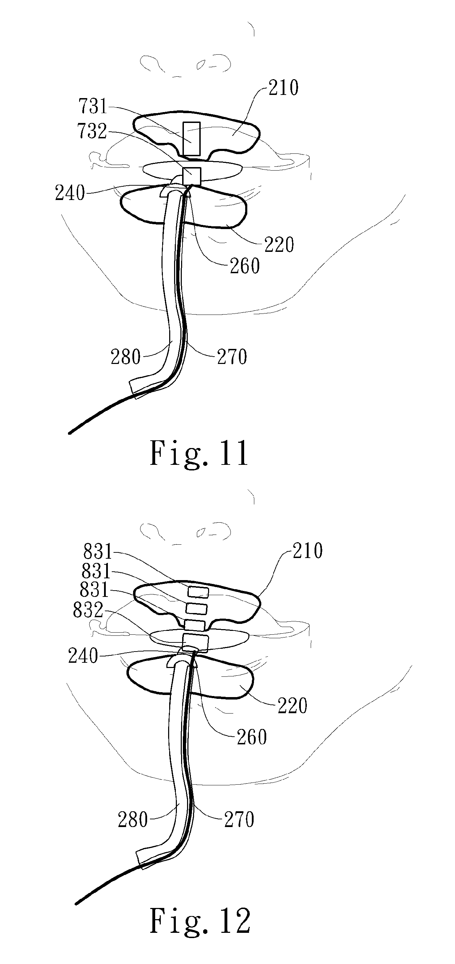

Fig. 11 is a schematic diagram of the oral apparatus according to Fig. 5A wherein the anchoring part 230 is a reclosable fastening part. The reclosable fastening part 230 may be fabric fasteners such as Dual Lock or Velcro tapes. Using Velcro tape as an example, the reclosable fastening part 230 may comprise a tiny-hooks part 731 and a hairy-loops part 732. The tiny-hooks part 731 can be installed and fixed on the upper attaching component 210, and the hairy-loops part 732 can be connected to the connecting part 240. The tiny-hooks part 731 and the hairy-loops part 732 can be attached and detached, which allows a user to separate the reclosable fastening part temporarily and manually to let the user to open mouth to drink, speak or perform other activities. Besides, the tiny-hooks part 731 can be attached onto different locations of the hairy-loops part 732 to allow for anatomical difference. It can be adjusted by users to accommodate thicker or thinner lips or provide tighter or looser mouth closing as desired.

Fig. 12 is a schematic diagram of the oral apparatus according to Fig. 5A wherein the anchoring part 230 is a lasso-pole anchoring part. The lasso-pole anchoring part 230 comprises a pole part 831 and a lasso part 832. The pole part 831 may have multiple pole structures. The pole part 831 can be installed and fixed on the upper attaching component 210, and the lasso part 832 can be connected to the connecting part 240. The pole part 831 and the lasso part 832 can be hooked and unhooked, which allows a user to separate the lasso-pole anchoring part temporarily and manually to let the user to open mouth to drink, speak or perform other activities. Besides, the lasso part 832 can be hooked onto different locations of the pole part 831 to allow for anatomical difference. It can be adjusted by users to accommodate thicker or thinner lips or provide tighter or looser mouth closing as desired.

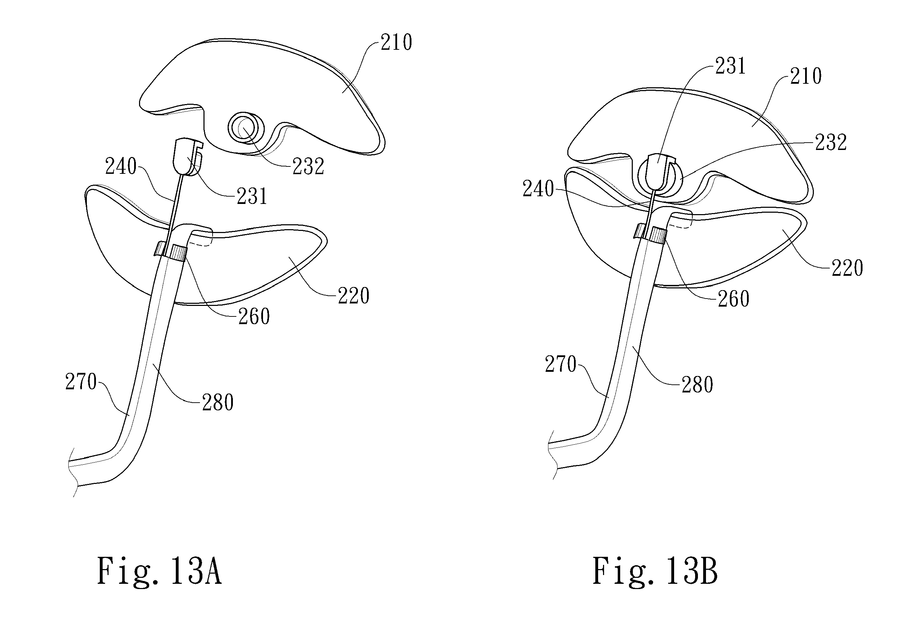

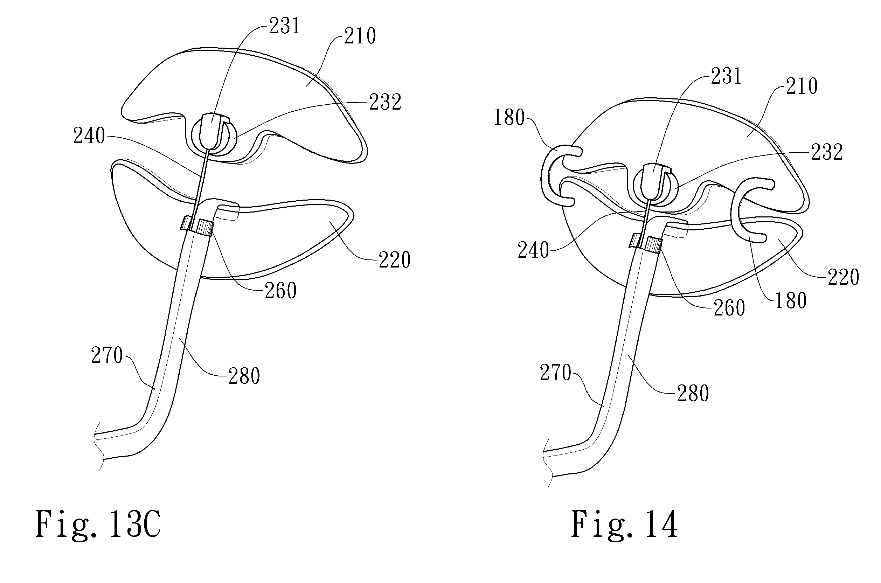

Please refer to Fig. 13A, Fig. 13B, and Fig. 13C to further understand the structure of the oral apparatus.

Fig. 13A is a perspective diagram of the oral apparatus according to Fig. 6, wherein the oral apparatus is in a disengaged condition. Fig. 13B is a perspective diagram of the oral apparatus according to Fig. 6 wherein the oral apparatus is in an engaged condition with a user's mouth being closed. Fig. 13C is a perspective diagram of the oral apparatus according to Fig. 6, wherein the oral apparatus is in an engaged condition with a user's mouth being open. As mentioned above, the oral apparatus can further comprise an extensible link(s) 180. The extensible link 180 is flexible and can connect the upper attaching component 210 and the lower attaching component 220 to prevent loss of any of the attaching components due to separation, as shown in Fig. 14.

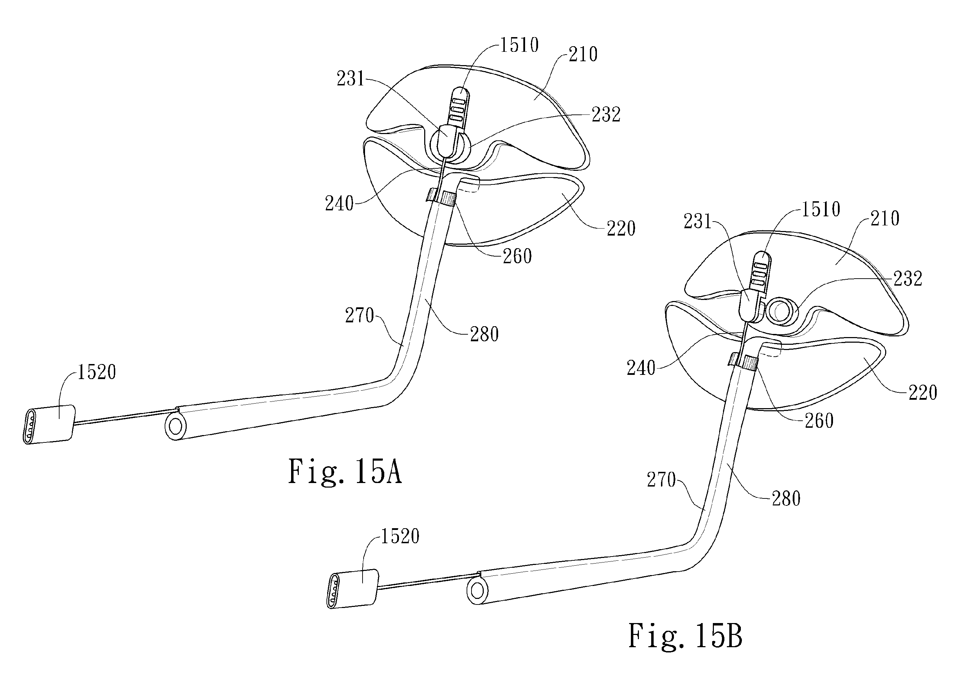

Fig. 15A is a variation of the oral apparatus according to Fig. 13A further comprising a sensor to detect a user's physiological or other conditions. The oral apparatus may further comprise a sensor component 1510 to detect a user's body conditions, such as a user's flow conditions of nasal breathing in order to judge if the user's breathing pattern is normal, with difficulty, or with pauses, etc. In one preferred embodiment, the connecting part 240 may further comprise a sensor component 1510 with sensing capability and electrical connection to deliver signal of body conditions sensed by the sensor component 1510 to a medical system (not shown) or the controller 251 (not shown in Fig. 15A and Fig. 15B). In another preferred embodiment of the present invention, the sensor component 1510 can be installed on the anchoring block 231.

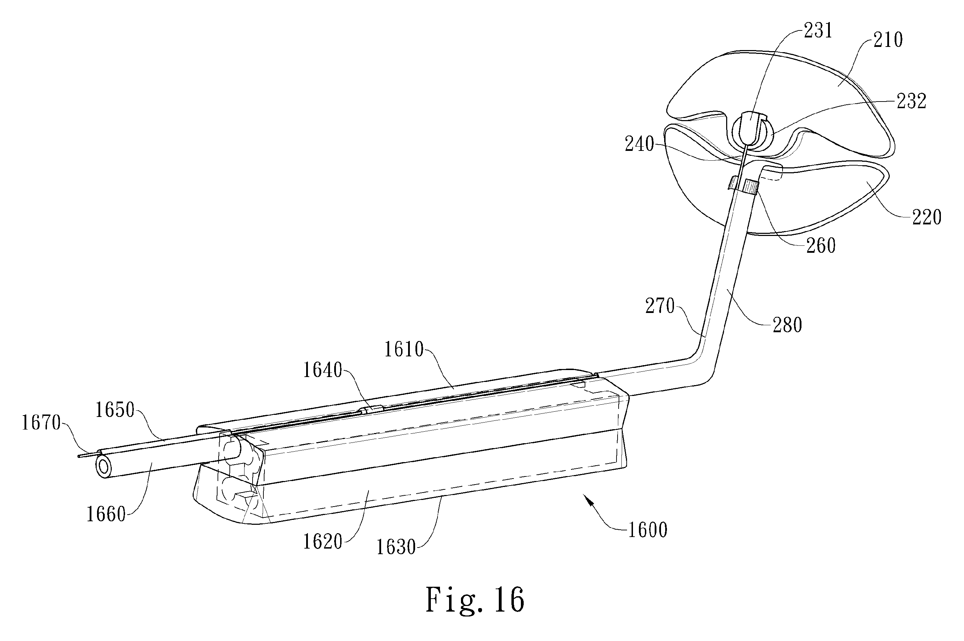

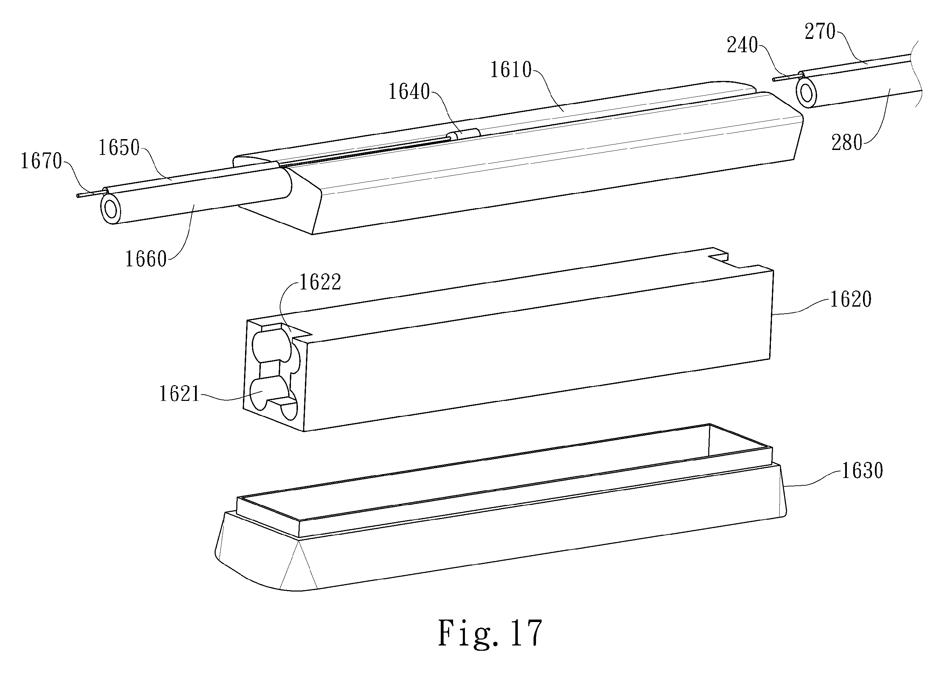

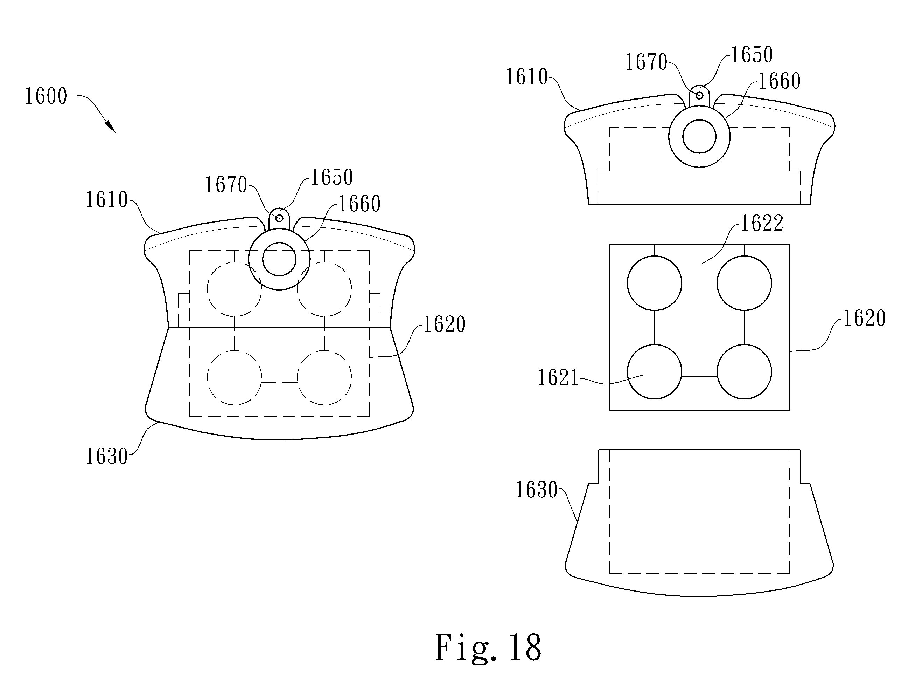

Fig. 16 is a variation of the oral apparatus according to Fig. 13A, further comprising a liquid canister 1600. The connecting part 240 and the fluid conduit 280 may connect to the retractable part 250 (not shown) and the negative pressure source 290 (not shown), respectively. The liquid canister 1600 may comprise a liquid chamber, a connector 1640, a fluid conduit 1660, and a connecting part 1670. The liquid chamber may comprise an upper lid 1610, a lower lid 1630 and a liquid absorber 1620. The liquid absorber 1620 may comprise one or more channels 1621 and/or spaces 1622 to maintain air flow patency within the liquid chamber even when the liquid absorber 1620 is absorbed with liquid. The fluid conduit 1660 connects to the negative pressure source 290 and the connecting part 1670 connects to the retractable part 250. Fig 17 shows an exploded view of the liquid chamber according to Fig. 16. The fluid conduit 280 can be coupled to one end of the liquid chamber to connect to the fluid conduit 1660. The connecting part 240 can be coupled with the connector 1640 to connect to the connecting part 1670. Therefore, the air conduit 280 can be connected to the negative pressure source 290 via the fluid conduit 1660, and the connecting part 240 can be connected to the retractable part 250 via the connecting part 1670. In one preferred embodiment, the liquid canister 1600 may further comprise a connecting catheter 1650 similar to the connecting catheter 270, in order to enclose and protect the connecting part 1670. Fig. 18 shows the liquid canister 1600, in which the left side shows a cross-section view of the liquid canister 1600 and the right side shows a fragmentarily cross-section view of the liquid canister 1600.

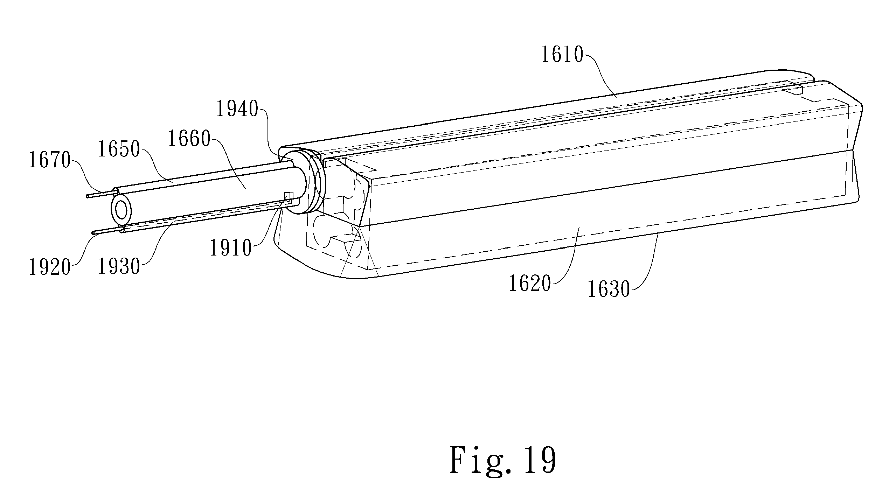

Fig. 19 shows another variation of the liquid canister 1600. The liquid canister 1600 may further comprise a filter 1940. The filter 1940 has a property to allow only gas to pass through it, thus when the liquid chamber is filled with saliva and water, the liquid cannot pass through the filter 1940. The condition of the liquid canister 1600 being filled with saliva or water can be determined by sensing the pressure or humidity in the fluid conduit 1660. The liquid canister 1600 may further comprise a pressure sensor 1910 installed in the fluid conduit 1660 or the liquid chamber to measure the pressure within the fluid conduit 1660 or the liquid chamber. The signal from the pressure sensor 1910 can be transmitted to an external system or the controller 251 via a sensing wire 1920 enclosed by a connecting catheter 1930. In another embodiment, the liquid canister 1600 may further comprise a humidity sensor 1910 installed in the fluid conduit 1660 or the liquid chamber to measure the humidity within the fluid conduit 1660 or the liquid chamber. The signal from the humidity sensor 1910 can be transmitted to an external system or the controller 251 via a sensing wire 1920 enclosed by a connecting catheter 1930.

In addition to the specific uses described above, other embodiments and uses of the invention will be apparent to those skilled in the art from consideration of the specification and practice of the invention disclosed herein. The specification and examples should be considered exemplary only with the scope of the invention indicated by the following claims. As will be easily understood by those of ordinary skill in the art, variations and modifications of each of the disclosed embodiments can be easily made within the scope of this invention as defined by the following claims.

## Claims

1. An oral apparatus for improving airway patency in a user, comprising:
an upper attaching component (110; 210) adapted to be adhered to regions near an upper lip of the user;
a lower attaching component (120; 220) adapted to be adhered to regions near a lower lip of the user;
a connecting part (140; 240) for connecting the upper (110; 210) and lower (120; 220) attaching components; and
a retractable part (150; 250) coupled to one end of the connecting part (140; 240);
the retractable part (150; 250) is used for controlling a distance between the upper (110; 210) and lower (120; 220) attaching components along a direction parallel to a direction from the upper lip toward the lower lip so as to close the user's mouth;
the connecting part (140) comprises a flexible wire, and said oral apparatus comprising an anchoring part (130; 230) disposed on the upper attaching component (110; 210) and connecting with the other end of the connecting part (140; 240).

2. The oral apparatus of claim 1, wherein the connecting part is adjustable with multiple connecting sub-parts (431, 831) to control the distance between the upper and lower attaching components.

3. The oral apparatus of claim 1, wherein the flexible wire (140) is connected to a fixed anchor (1501; 1508).

4. The oral apparatus of claim 1, wherein the retractable part is in one of the following form: piston-type (1502), bellows-type (1505), magnetic-type (1506, 1507), spring-type (1509), plate-type (1510), or self-retracting-type (1511).

5. The oral apparatus of claim 1, wherein the retractable part (250) further comprises an actuator sub-part (252).

6. The oral apparatus of claim 1, wherein the retractable part (250) further comprises a control sub-part (251).

7. The oral apparatus of claim 1, wherein at least one of the attaching components (210, 220) comprises at least one sensor (1510) to detect the user's physiological conditions and/or other conditions.

8. The oral apparatus of claim 1, further comprising a liquid conduit engaging to one of the attaching components to interface a negative pressure source and the user's oral cavity.

9. The oral apparatus of claim 8, further comprising a liquid canister (1600) with the liquid conduit (280) and the connecting part (240) passing therethrough.

10. The oral apparatus of claim 9, wherein the liquid canister (1600) further comprises a liquid absorber (1620) and/or a filter (1940).

11. The oral apparatus of claim 9, wherein the liquid canister (1600) further comprises a sensor (1910).

12. The oral apparatus of claim 10, wherein the liquid absorber (1620) is provided with one or more channels (1621) and/or spaces (1622).

13. The oral apparatus of claim 1, wherein the anchoring part (230) is selected from the following: mechanical connectors (231, 232), magnetic connectors (331, 332), male-and-female connector (431, 432), frictional connectors (531, 532), adhesive connectors (631, 632), non-adhesive fastener (731, 732), and loop-and-hook connectors (831, 832).

14. The oral apparatus of claim 7, wherein said sensor (1510) is adapted to detect a relative distance (d) between the upper and lower lips of the user's mouth and determines the user's mouth is open if the relative distance (d) is equal to or larger than a first distance (d1).

15. The oral apparatus of claim 14, wherein the retractable part (250) further comprises an actuator (252) is adapted to pull the connecting part (240) to reduce the distance between the upper and lower lips until said sensor (1510) detects the relative distance (d) is smaller than a second distance (d2) and determining the user's mouth is closed.

## Patentansprüche

1. Orale Vorrichtung zur Verbesserung der Durchgängigkeit der Wege eines Benutzers, umfassend:
eine obere Befestigungskomponente (110; 210), die zum Ankleben an Bereiche nahe der Oberlippe des Benutzers angepasst ist;
eine untere Befestigungskomponente (120; 220), die zum Ankleben an Bereiche nahe der Unterlippe des Benutzers angepasst ist;
ein Verbindungsteil (140; 240) zum Verbinden der oberen (110; 210) und unteren (120; 220) Befestigungskomponente; und
ein an ein Ende des Verbindungsteils (140; 240) gekoppeltes Rückzugsteil (150; 205);
das Rückzugsteil (150; 250) wird zum Kontrollieren eines Abstands zwischen dem oberen (110; 210) und unteren (120; 220) Befestigungsteil längs einer Richtung, die parallel zu einer von der Oberlippe zur Unterlippe verlaufenden Richtung verläuft, verwendet, um den Mund des Benutzers zu schließen,
das Verbindungsteil (140) umfasst ein flexible Leitung, und
die orale Vorrichtung umfasst ein an der oberen Befestigungskomponente (110; 210) angeordnetes und mit dem anderen Ende des Verbindungsteils (140; 240) verbindendes Verankerungsteil (130; 230).

2. Orale Vorrichtung nach Anspruch 1, wobei das Verbindungsteil mit mehreren Verbindungs-Unterteilen (431, 831) zur Kontrolle des Abstands zwischen der oberen und unteren Befestigungskomponente anpassbar ist.

3. Orale Vorrichtung nach Anspruch 1, wobei die Leitung (140) mit einem Befestigungsanker (1501; 1508) verbunden ist.

4. Orale Vorrichtung nach Anspruch 1, wobei das Rückzugsteil von einer der folgenden Formen ist: Kolbentyp (1502), Faltenbalgtyp (1505), Magnettyp (1506, 1507), Federtyp (1509), Plattentyp (1510) oder Selbst-Zurückziehender Typ (1511).

5. Orale Vorrichtung nach Anspruch 1, wobei das Rückzugsteil (250) weiter ein Aktuator-Unterteil (252) umfasst.

6. Orale Vorrichtung nach Anspruch 1, wobei das Rückzugsteil (250) weiter ein Kontroll-Unterteil (251) umfasst.

7. Orale Vorrichtung nach Anspruch 1, wobei mindestens eine der Befestigungskomponenten (210, 220) mindestens einen Sensor (1510) zur Detektion des physiologischen Zustands und/oder anderer Zustände des Benutzers umfasst.

8. Orale Vorrichtung nach Anspruch 1, weiter umfassend eine ein Ende der Befestigungskomponenten in Eingriff nehmende Flüssigkeitsleitung zum Koppeln einer Unterdruckquelle und der Mundhöhle des Benutzers.

9. Orale Vorrichtung nach Anspruch 8, weiter umfassend einen Flüssigkeitsbehälter (1600), durch den die Flüssigkeitsleitung (180) und das Befestigungsteil (42) durchreichen.

10. Orale Vorrichtung nach Anspruch 9, wobei der Flüssigkeitsbehälter (1600) weiter einen Flüssigkeitsabsorber (1620) und/oder einen Filter (1940) umfasst.

11. Orale Vorrichtung nach Anspruch 9, wobei der Flüssigkeitsbehälter (1600) weiter einen Sensor (1910) umfasst.

12. Orale Vorrichtung nach Anspruch 10, wobei der Flüssigkeitsabsorber (16 mit einem oder mehreren Kanälen (16) und/oder Räumen (1622) versehen ist.

13. Orale Vorrichtung nach Anspruch 1, wobei das Ankerteil (230) aus einem der folgenden ausgewählt ist: mechanische Verbinder (231, 232), magnetische Verbinder (331, 332), Männchen-und-Weibchen-Verbinder (431, 432), Reibungsverbinder (531, 532), klebende Verbinder (631, 632), nicht-klebende Befestigungselemente (731, 732) und Klettverschlussverbinder (831,832).

14. Orale Vorrichtung nach Anspruch 7, wobei der Sensor (1510) zur Detektion eines relativen Abstands (d) zwischen der Oberlippe und der Unterlippe des Mundes des Benutzers angepasst ist und bestimmt, ob der Mund des Benutzers offen ist, falls der relative Abstand (d) gleich oder größer als ein erster Abstand (d1) ist.

15. Orale Vorrichtung nach Anspruch 14, wobei das Rückzugsteil (250) weiter umfasst einen Aktuator (252), der zum Ziehen des Verbindungsteils (240) angepasst ist, um den Abstand der Oberlippe und Unterlippe zu verringern, bis der Sensor (1510) detektiert, dass der relative Abstand (d) kleiner ist als ein zweiter Abstand (d2), und bestimmt, dass der Mund des Benutzers geschlossen ist.

## Revendications

1. Dispositif oral destiné à améliorer la capacité des voies respiratoires d'un utilisateur, comprenant :
un composant de fixation supérieur (110 ; 210) adapté de manière à adhérer sur des zones proches d'une lèvre supérieure de l'utilisateur ;
un composant de fixation inférieur (120 ; 220) adapté de manière à adhérer sur des zones proches d'une lèvre inférieure de l'utilisateur ;
une partie de liaison (140 ; 240) destinée à relier les composants de fixation supérieur (110 ; 210) et inférieur (120 ; 220) ; et
une partie rétractable (150 ; 250) couplée à une première extrémité de la partie de liaison (140 ; 240) ;
la partie rétractable (150 ; 250) est utilisée de manière à commander une distance entre les composants de fixation supérieur (110 ; 210) et inférieur (120 ; 220) suivant une direction parallèle à une direction partant de la lèvre supérieure vers la lèvre inférieure afin de fermer la bouche de l'utilisateur ;
la partie de liaison (140) comprend un câble flexible, et
ledit dispositif oral comprend une partie d'ancrage (130 ; 230) disposée sur le composant de fixation supérieur (110 ; 210) et reliée à l'autre extrémité de la partie de liaison (140 ; 240).

2. Dispositif oral selon la revendication 1, dans lequel la partie de liaison peut être réglée avec de multiples composants de liaison secondaires (431, 831) afin de commander la distance entre les composants de fixation supérieur et inférieur.

3. Dispositif oral selon la revendication 1, dans lequel le câble flexible (140) est relié à un élément d'ancrage fixe (1501 ; 1508).

4. Dispositif oral selon la revendication 1, dans lequel la partie rétractable se présente sous l'une des formes suivantes : du type piston (1502), du type soufflet (1505), du type magnétique (1506, 1507), du type à ressort (1509), du type en plaque (1510), ou du type auto-rétractable (1511).

5. Dispositif oral selon la revendication 1, dans lequel la partie rétractable (250) comprend, en outre, une partie d'activation secondaire (252).

6. Dispositif oral selon la revendication 1, dans lequel la partie rétractable (250) comprend, en outre, une partie de commande secondaire (251).

7. Dispositif oral selon la revendication 1, dans lequel au moins l'un des composants de fixation (210, 220) comprend au moins un capteur (1510) afin de détecter les conditions physiologiques de l'utilisateur et/ou d'autres conditions.

8. Dispositif oral selon la revendication 1, comprenant, en outre, un conduit de liquide couplé à l'un des composants de fixation afin d'assurer l'interface entre une source de dépression et la cavité orale de l'utilisateur.

9. Dispositif oral selon la revendication 8, comprenant, en outre, un collecteur de liquide (1600) à travers lequel passent le conduit de liquide (280) et la partie de liaison (240).

10. Dispositif oral selon la revendication 9, dans lequel le collecteur de liquide (1600) comprend, en outre, un élément d'absorption de liquide (1620) et/ou un filtre (1940).

11. Dispositif oral selon la revendication 9, dans lequel le collecteur de liquide (1600) comprend, en outre, un capteur (1910).

12. Dispositif oral selon la revendication 10, dans lequel l'élément d'absorption de liquide (1620) comporte un ou plusieurs canaux (1621) et/ou espaces (1622).

13. Dispositif oral selon la revendication 1, dans lequel la partie d'ancrage (230) est sélectionnée parmi les suivants : des éléments de liaison mécaniques (231, 232), des éléments de liaison magnétiques (331, 332), des éléments de liaison mâle et femelle (431, 432), des éléments de liaison à friction (531, 532), des éléments de liaison adhésifs (631, 632), des éléments de liaison non adhésifs (731, 732) et des éléments de liaison à boucle et crochet (831, 832).

14. Dispositif oral selon la revendication 7, dans lequel ledit capteur (1510) est adapté afin de détecter une distance relative (d) entre les lèvres supérieure et inférieure de la bouche de l'utilisateur et détermine que la bouche de l'utilisateur est ouverte si la distance relative (d) est supérieure ou égale à une première distance (d1).

15. Dispositif oral selon la revendication 14, dans lequel la partie rétractable (250) comprend, en outre, un actionneur (252) adapté de manière à entraîner la partie de liaison (240) afin de réduire la distance entre les lèvres supérieure et inférieure jusqu'à ce que ledit capteur (1510) détecte que la distance relative (d) est inférieure à la seconde distance (d2) et détermine que la bouche de l'utilisateur est fermée.
